Europäisches Patentamt

European Patent Office

Office européen des brevets

(19)

(11) Veröffentlichungsnummer: **0 400 459**
**A1**

## (12) EUROPÄISCHE PATENTANMELDUNG

(21) Anmeldenummer: 90109693.3

(22) Anmeldetag: 22.05.90

(51) Int. Cl.⁵: **B65B 55/10, A61L 2/04, A23L 3/10**

(30) Priorität: 29.05.89 DE 8906572 U

(43) Veröffentlichungstag der Anmeldung:
05.12.90 Patentblatt 90/49

(84) Benannte Vertragsstaaten:
AT CH DE DK FR IT LI

(71) Anmelder: MMM MÜNCHENER MEDIZIN
MECHANIK GMBH
Halmstrasse 6
D-8000 München 70(DE)

(72) Erfinder: Kammermeier, Bernhard, Dipl.-Ing.
Thalkirchner Strasse 188
D-8000 München 70(DE)

(74) Vertreter: Patentanwälte Grünecker,
Kinkeldey, Stockmair & Partner
Maximilianstrasse 58
D-8000 München 22(DE)

(54) **Berieselungsanordnung zur Berieselung von Gütern in einem Beladeraum.**

(57) Es wird eine Berieselungsanordnung (1) zur Berieselung von Gütern (13) in einem Beladeraum (5) vorgeschlagen, die etagenweise übereinander auf Beladeebenen (11) angeordnet sind, wobei Berieselungsflüssigkeitszuführungen zur Abgabe der Berieselungsflüssigkeit (24) an die Güter (13) über eine Lochplatte (19) vorgesehen sind. Die Berieselungsanordnung (1) kennzeichnet sich dadurch aus, daß oberhalb jeder Beladeebene (11) eine dieser individuell zugeordnete Lochplatte (19) vorgesehen ist, daß außerhalb des Beladeraums (5) für die Berieselungsflüssigkeit (24) jeder Beladeebene (11) gesondert zuführende erste Strömungswege vorgesehen sind, die jeweils die der Beladeebene (11) zugeordnete Lochplatte (19) mit für jede Beladeebene (11) mit gleicher Füllmenge beaufschlagen und das oberhalb jeder Lochplatte (19) mit Ausnahme der obersten (33) und untersten (34) Lochplatte ein die Berieselungsflüssigkeit (24) in im wesentlichen horizontaler Richtung zu wenigstens einer Seite des Beladeraums (5) hin abführender zweiter Strömungsweg vorgesehen ist.

FIG.1

## Berieselungsanordnung zur Berieselung von Gütern in einem Beladeraum

Die vorliegende Erfindung betrifft eine Berieselungsanordnung zur Berieselung von Gütern in einem Beladeraum, nach dem Oberbegriff des Anspruchs 1.

Es sind Berieselungssysteme bekannt, bei denen das zu berieselnde Gut durch im Beladeraum seitlich angeordnete Düsen bespritzt wird. Diese seitlichen Düsen sind mit einem Rohrsystem verbunden, durch das die Berieselungsflüssigkeit an die Düsen herangeführt wird. Ein wesentlicher Nachteil dieses Systems ist, daß die Berieselung der Güter, beispielsweise Flaschen, nur unzureichend bzw. ungenau erfolgt, so daß eine gleichmäßige Beaufschlagung der Güter mit Berieselungswasser, beispielsweise zum Zwecke der Sterilisation, nicht erfolgt. Des weiteren ergibt sich gemäß diesem Stand der Technik der Nachteil, daß die Düsen fest angeordnet sind, so daß Änderungen der Beladeebenen in vertikaler Richtung zur Anpassung an unterschiedliche Größen des zu berieselnden Gutes zu noch größeren Ungleichmäßigkeiten der Berieselung führen können.

Es ist weiterhin bekannt, die Berieselungsflüssigkeitszuführungen dadurch vorzusehen, daß die zu berieselnden Güter auf Beladeebenen ruhen, die als Lochbleche ausgebildet sind, wobei die von oben herabrieselnde Berieselungsflüssigkeit entlang der Güter strömt, auf die Lochbleche auftrifft und durch die Löcher weiter nach unten auf die nächste mit Gütern bestückte Beladeebene fällt. Der wesentliche Nachteil dieser Berieselungsanordnung besteht darin, daß keine gleichmäßige Berieselung aller Güter sowohl hinsichtlich Menge als auch Temperatur gewährleistet ist. Dies hat insbesondere bei der Berieselung zum Zwecke der Sterilisierung der Güter den Nachteil, daß nicht gewährleistet ist, daß alle Güter entsprechend ausreichend berieselt werden und somit eine sichere Sterilisation nicht gegeben ist. Dies rührt daher, daß durch Wärmeaustausch in der obersten Etage die Berieselungsflüssigkeit bereits Temperatur an das Sterilisierungsgut abgibt, so daß die Temperatur bereits in der nächstfolgenden Etage nicht mehr mit der Temperatur der ersten Etage übereinstimmt. Somit stellt sich der Temperaturverlust bzw. bei Kühlung der Temperaturanstieg von Etage zu Etage fort, so daß keine gleichmäßigen Verhältnisse in dem Beladeraum vorherrschen.

Die Gleichmäßigkeit der Verteilung der Berieselungsflüssigkeit wird weiterhin dadurch noch beeinträchtigt, daß durch das Gut abgedeckte Löcher der Lochbleche keine Berieselungsflüssigkeit an die nächste Ebene weitergeben können, so daß darunter ein "Berieselungsschatten" entsteht und das darunterstehende Gut u.U. überhaupt nicht mit Berieselungsflüssigkeit beaufschlagt wird.

Schließlich ist bei dichtgedrängter Aufstellung, welche für eine entsprechende Ausnutzung des Beladeraums vorteilhaft ist, die Versorgung von mehreren Etagen über Berieselungsbleche praktisch unmöglich, da keine ausreichende Flüssigkeitsmenge von Etage zu Etage weiterfließt.

Es ist deshalb Aufgabe der vorliegenden Erfindung, eine Berieselungsanordnung der eingangs genannten Art zu schaffen, bei der eine gleichmäßige Verteilung der Berieselungsflüssigkeit über alle Etagen ermöglicht wird, so daß weiterhin Temperaturunterschiede zwischen den einzelnen berieselten Etagen nahezu nicht mehr vorhanden sind.

Diese Aufgabe wird erfindungsgemäß dadurch gelöst, daß oberhalb jeder Beladeebene eine dieser individuell zugeordnete Lochplatte vorgesehen ist, daß außerhalb des Beladeraums die Berieselungsflüssigkeit jeder Beladeebene gesondert zuführende erste Strömungswege vorgesehen sind, die jeweils die der Beladeebene zugeordnete Lochplatte mit für jede Beladeebene gleicher Füllmenge beaufschlagen, und daß oberhalb jeder Lochplatte mit Ausnahme der obersten und untersten ein die Berieselungsflüssigkeit in im wesentlichen horizontaler Richtung zu wenigstens einer Seite des Beladeraums hin abführender zweiter Strömungsweg vorgesehen ist.

Dadurch wird erreicht, daß das sich auf jeder Beladeebene befindende Gut gleichmäßig berieselt wird, und sich kein oder nahezu kein Temperaturunterschied zwischen der obersten Berieselungsebene und der untersten Berieselungsebene einstellt, da insbesondere die Berieselungsflüssigkeit, die eine Etage beaufschlagt, nicht mehr in Berührung mit dem Gut anderer Etagen kommt.

Somit wird vorteilhafterweise erreicht, daß jede Beladeebene gleichmäßig sowohl vom Durchsatz als auch der Temperatur von der Berieselungsflüssigkeit beaufschlagt wird, so daß im gesamten Beladeraum konstante bzw. annähernd konstante Bedingungen' herrschen. Somit wird gewährleistet, daß das gesamte sich im Beladeraum befindliche Gut gleichmäßig behandelt werden kann.

Vorteilhafterweise ergibt sich weiterhin, daß die erfindungsgemäße Berieselungsanordnung einfach aufgebaut ist, und das zu berieselnde Gut auch sehr eng aneinander angeordnet werden kann, was zu einer besonders vorteilhaften Ausnutzung des Beladeraums führt.

Weitere Vorteile ergeben sich aus den Unteransprüchen.

Gemäß einer bevorzugten Ausführungsform weist die Berieselungsanordnung eine Kammer auf, in die ein die Beladeebenen aufweisender Wagen einbringbar ist. Der erste Strömungsweg ist hierbei durch innere und äußere Leitelemente begrenzt, was vorteilhafterweise zu einem besonders einfachen Aufbau führt.

Wenn die Leitelemente sich dabei im wesentlichen in vertikaler Richtung erstrecken, wird eine besonders einfache Berieselungsflüssigkeitsführung erreicht.

Eine besonders einfache und wirkungsvolle Ausgestaltung des ersten Strömungswegs wird dadurch erreicht, daß die inneren Leitelemente an ihren unteren Enden mit der zugeordneten Lochplatte verbunden sind und gegenüber der Vertikalen leicht geneigt angeordnet sind. Dadurch kann die von oben auftreffende Berieselungsflüssigkeit auf einfachste Weise der entsprechenden Lochplatte der zugehörigen Beladeebene zugeführt werden.

Werden weiterhin die äußeren und/oder die inneren Leitelemente am Wagen angebracht, so ergibt sich vorteilhafterweise, daß eine besonders kompakte Bauweise der Berieselungsanordnung ermöglicht wird, wobei insbesondere entsprechende Anpassungsmöglichkeiten der Beladeebene möglich sind.

Dadurch, daß die äußeren Leitelemente in Strömungsrichtung der Berieselungsflüssigkeit nach innen vorspringen, wird erreicht, daß eine gezielte Zuführung der Berieselungsflüssigkeit von einer Beladeebene zur anderen ermöglicht wird, wodurch zuverlässig jede Beladeebene mit ausreichender Berieselungsflüssigkeit versorgt werden kann.

Durch die Verstellbarkeit der Beladeebenen in vertikaler Richtung wird eine besonders anpassungsfähige Berieselungsanordnung an jegliche Art zu berieselnder Güter geschaffen, wobei insgesamt vorteilhaft unterschiedlichste Abstände der einzelnen Beladeebenen bei gleichzeitiger Beibehaltung der konstanten Berieselungsbedingungen möglich sind.

Dieser Vorteil wird weiterhin dadurch sichergestellt, daß die Berieselungsflüssigkeitsmenge und der Lochquerschnitt der Löcher der Lochplatten so aufeinander abgestimmt sind, daß eine von Beladeebene zu Beladeebene abnehmende Berieselungsflüssigkeitsmenge überläuft und sich bevorzugt in der untersten Beladeebene ein konstanter Berieselungsflüssigkeitspegel einstellt. Dadurch wird vorteilhafterweise erreicht, daß einerseits überschüssige Berieselungsflüssigkeitsmengen vermieden werden können, welche insbesondere bei der Sterilisation durch ihren erhöhten Druck und erhöhte Temperatur entsprechende Energieaufwendungen benötigen, so daß andererseits entsprechende Einsparungen beim Umwälzen der erheblichen Flüssigkeitsmengen möglich sind.

Dadurch, daß die oberste Lochplatte von einer Platte nahezu vollständig überdeckt ist und der Flüssigkeitseintritt an einer gegenüberliegenden Seite erfolgt, wird eine besonders gute Verteilung der frisch eintretenden Berieselungsflüssigkeit erzeugt, und wird sichergestellt, daß alle Beladeebenen unter praktisch den gleichen Bedingungen beaufschlagt werden.

In einer bevorzugten Ausführungsform ist die Beladeebene, auf der die Güter ruhen, aus einer wellenförmigen oder zick-zack-förmigen Platte gebildet, so daß die Berieselungsflüssigkeit schnell und ohne Behinderung abgeleitet werden kann. Dies wird dadurch noch besonders gut verwirklicht, wenn die Berieselungsflüssigkeit quer zur Eintritts- bzw. Fallrichtung abgeführt wird. Hierzu verläuft das Profil der wellen- oder zick-zack-förmigen Platte entlang den Längsseiten der Kammern, d.h. die Wellen- oder Zick-Zack-Form erstreckt sich quer zu den Längsseiten.

Um schwere Güter aufnehmen zu können, weist die erfindungsgemäße Berieselungsanordnung unterhalb der profilierten Platte eine im wesentlichen ebene Versteifungsplatte auf.

Zur Erhöhung der Traglast der Beladeebene kann weiterhin eine im wesentlichen ebene Versteifungsplatte auch oberhalb der profilierten Platte vorgesehen werden, die dann zur Abführung der Berieselungsflüssigkeit mit entsprechenden Ausnehmungen, Löchern oder Schlitzen im Bereich der Senken der Profils der profilierten Platte versehen ist.

Die Gleichmäßigkeit des Berieselungsvorgangs wird dadurch noch erhöht, daß die abfließende Berieselungsflüssigkeit an den Stirnseiten der Beladeebenen einen Wasservorhang bildet. Dadurch wird insbesondere ein Wärmeverlust an den Endseiten des Beladeraums vermieden und somit eine Gleichmäßigkeit der Temperatur über den gesamten Beladeraum erzeugt.

Vorteilhafterweise kann die Zuführung der Berieselungsflüssigkeit auch mit Überlaufbehältern bzw. Aufnahmeräumen versehen sein, in denen Überlaufstutzen angeordnet sind, die ein Ablaufen der Berieselungsflüssigkeit nach unten erlauben. Werden dabei die Überlaufstutzen einer Beladeebene bezüglich den benachbarten Beladeebenen versetzt angeordnet, so wird erreicht, daß ein direktes Durchfließen der von oben herabfallenden Berieselungsflüssigkeit vermieden wird und die Zuführung der Berieselungsflüssigkeit zu der zugeordneten Lochplatte sichergestellt ist.

Wenn die Verstellbarkeit der Beladeebenen durch Stangen erreicht wird, auf denen die Beladeebenen ruhen, und die Stangen ihrerseits in Löchern von Eckpfosten des Wagens gelagert werden, so wird auf einfachste Weise eine Höhenanpassung der Etage bzw. Beladeebene erreicht, ohne daß Werkzeuge zu deren Verstellbarkeit notwendig sind. Dies erlaubt eine schnelle Anpassung an die jeweilige Charge, so daß insgesamt ein erheblich schnellerer Durchsatz der Berieselungsanordnung erreicht wird.

Gemäß einer bevorzugten Ausführungsform sind die Beladeebenen seitlich schwenkbar angeordnet, so daß die Beschickung des Wagens einfach und somit sehr zeitsparend erfolgen kann.

Weitere Einzelheiten, Merkmale und Vorteile der vorliegenden Erfindung ergeben sich aus der nachfolgenden Beschreibung unter Bezugnahme auf die Zeichnung. Darin zeigt:

Fig. 1 eine vereinfachte, prinzipielle Darstellung der erfindungsgemäßen Berieselungsanordnung von der Stirnseite der Berieselungskammer aus gesehen;

Fig. 2 eine vereinfachte Prinzipdarstellung einer Berieselungskammer in Seitenansicht mit drei darin angeordneten Wagen;

Fig. 3 eine alternative Ausführungsform eines Teils des ersten Strömungswegs der erfindungsgemäßen Berieselungsanordnung;

Fig. 4 die Darstellung von quadratischem und kreisförmigem Ladegut auf eine Beladeebene; und

Fig. 5 einen Wagen mit drei Beladeebenen in hochgeschwenktem Zustand.

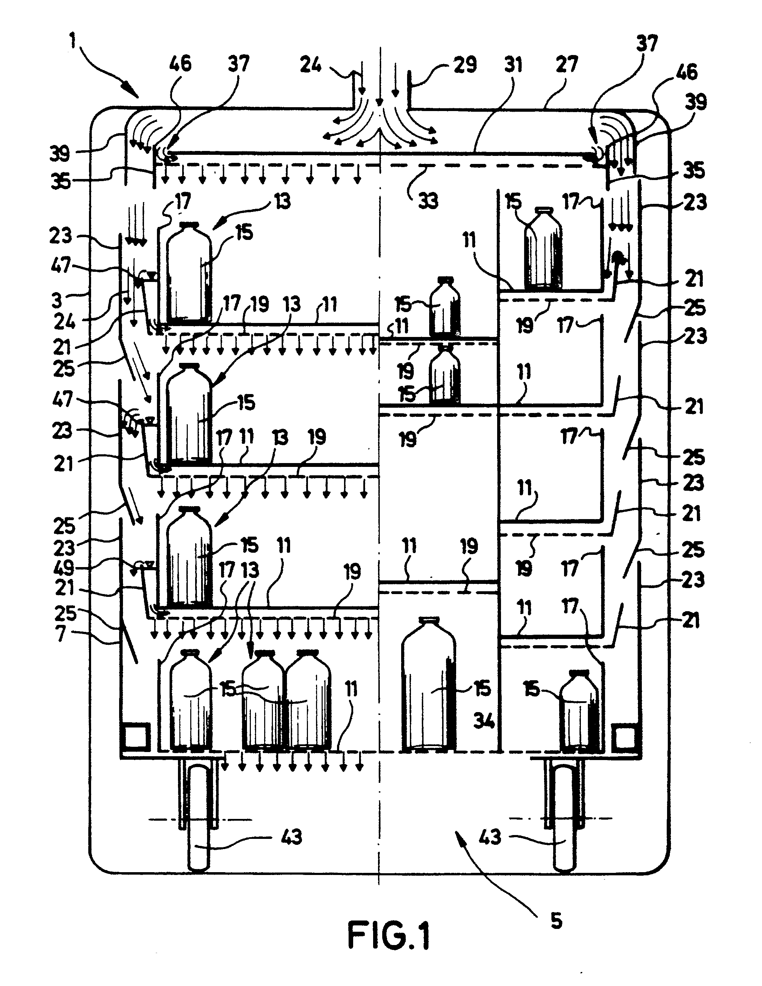

In Fig. 1 ist in vereinfachter Darstellung der prinzipielle Aufbau einer Berieselungsanordnung 1 dargestellt. Die Berieselungsanordnung 1 weist eine Kammer 3 auf, die einen Beladeraum 5 bildet. In dem Beladeraum 5 ist ein Wagen 7 angeordnet.

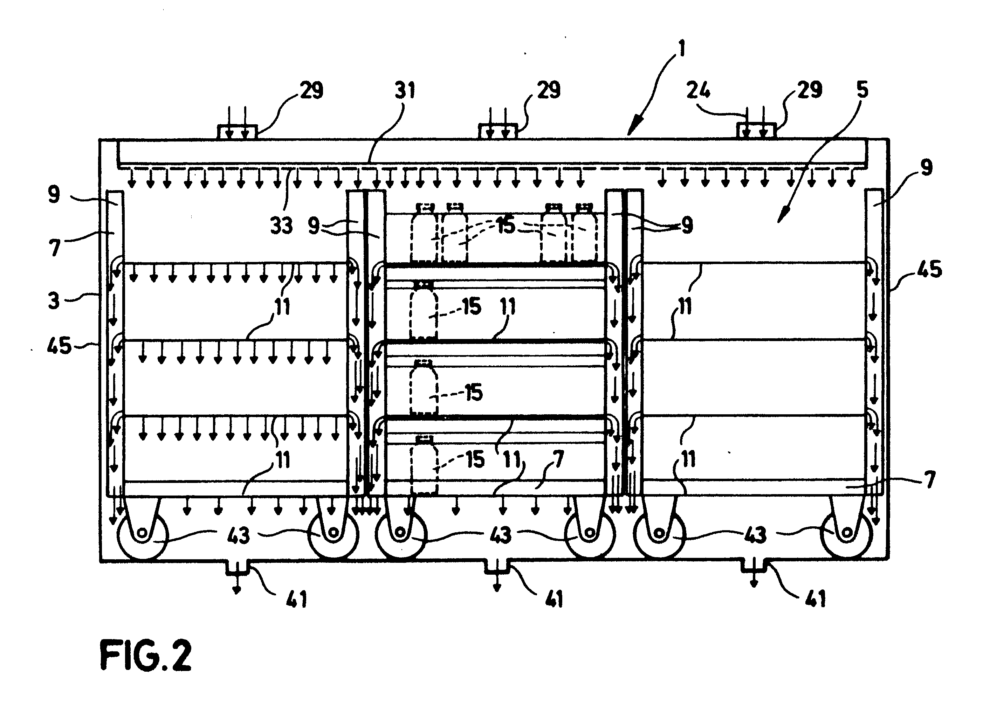

Der Wagen 7, wie deutlicher aus Fig. 2 ersichtlich, weist an seinen vier Ecken Eckpfosten 9 auf, die als Stützelemente für Beladeebenen 11 dienen. Die Beladeebenen 11 sind im einfachsten Fall als ebene Platten ausgebildet, auf denen das zu berieselnde Gut 13 angeordnet ist. Gemäß der Darstellung der linken Hälfte von Fig. 1 und der Darstellung gemäß Fig. 2 weisen die Beladeebenen 11 gleichen oder annähernd gleichen Abstand auf, und sind mit gleichgroßen Flaschen 15 bestückt. Die Beladeebenen 11 weisen in Wagenlängsrichtung seitliche Trennwände 17 auf, die mit den Beladeebenen 11 verbunden sind. Die Trennwände 17 verlaufen vorzugsweise in vertikaler Richtung. Oberhalb einer jeden Beladeebene 11 ist eine vorzugsweise ebene Lochplatte 19 angeordnet. Die Lochplatte 19 stößt an den Längsseiten des Wagens leicht über die Beladeebenen 11 hervor und weist an ihren Längsrändern innere Leitelemente 21 auf, die sich von der Lochplatte nach oben erstrecken. Die Leitelemente 21 können entweder vertikal oder bevorzugt leicht nach außen geneigt angeordnet sein. Weiterhin sind äußere Leitelemente 23 vorgesehen, die zusammen mit den Trennwänden 17 bzw. den inneren Leitelementen 21 einen ersten Strömungsweg für die Berieselungsflüssigkeit bilden.

Die äußeren Leitelemente 23 weisen untere Abschnitte 25 auf, die in Fließrichtung leicht nach innen geneigt angeordnet sind, um somit der von oben heranströmenden Berieselungsflüssigkeit 24 eine Richtungsänderung in Richtung auf die Beladeebenen 11 zu geben.

Die inneren und äußeren Leitelemente 21 und 23 können als Platten ausgebildet sein, die sich über die gesamte Längsseite des Wagens erstrekken. In einer bevorzugten Ausführungsform sind nicht nur die inneren Leitelemente 21, sondern auch die äußeren Leitelemente 23 am Wagen 7 befestigt, so daß hierbei die Möglichkeit gegeben wird, bei Verändern des Abstandes der Beladeebenen 11 untereinander den zugehörigen ersten Strömungsweg durch ebenfalls Versetzen der Leitelemente optimal anzupassen. In der rechten Hälfte von Fig. 1 sind verschiedene Anordnungsmöglichkeiten der Beladeebenen dargestellt, wobei der Aufbau prinzipiell jenem der linken Hälfte entspricht. Entsprechend sind auch die gleichen Bezugszeichen angegeben.

An der Oberseite 27 der Kammer 3 sind Einfüllstutzen 29 vorgesehen, durch die die Berieselungsflüssigkeit 24 in die Kammern 3 eingeführt wird. Unterhalb der Einfüllstutzen, beispielsweise ist ein Einfüllstutzen 29 pro Wagen (Fig. 2) vorgesehen, ist eine obere Platte 31 angeordnet, die, wie aus Fig. 2 ersichtlich, nahezu die gesamte Länge der Kammer 3 in ihrer Längsrichtung durchsetzt. Unterhalb der oberen Platte 31 ist eine Lochplatte 33 vorgesehen, die bezüglich der oberen Platte 31 hervorspringt und mit inneren Leitelementen 35 versehen ist, die mit den seitlichen Rändern der Lochplatte 33 verbunden sind. Die inneren Leitelemente 35 verlaufen exakt oder annähernd in vertikaler Richtung sowohl oberhalb als auch unterhalb der Lochplatte 33, so daß ein Abstand oder Zwischenraum 37 zwischen der oberen Platte 31 und den inneren Leitelementen 35 vorhanden ist. Weiterhin sind von der Kammeroberseite 27 nach unten sich erstreckende äußere Leitelemente 39 vorgesehen, die den eintretenden Berieselungsflüssigkeitsstrom nach unten ableiten.

Wie aus Fig. 2 ersichtlich, sind im Bodenbereich der Kammer 3 Ablaufstutzen 41 vorgesehen, die die von oben herabfallende Berieselungsflüssigkeit aus der Kammer 3 wieder herausführen.

Jeder Wagen 7 ist vorzugsweise auf vier Rädern 43 gelagert, wobei vorzugsweise zwei Räder einer Achse eines Wagens lenkbar sind.

Die Kammer 3 ist an den Stirnseiten durch entsprechende Tore 45 verschließbar, wobei auch die Möglichkeit vorhanden ist, nur an einer Seite der Kammer ein Tor vorzusehen.

Im Nachfolgenden wird nunmehr die Wirkungsweise der in den Fig. 1 und 2 dargestellten Berieselungsanordnung erläutert. Die Berieselungsflüssigkeit 24 wird durch den oder die Einfüllstutzen 29 in die Kammer 3 bzw. in den Beladeraum 5 eingebracht und stößt dabei zunächst auf die obere Platte 31. Von dieser wird sie zu den Längsseiten der Kammer abgeführt, wobei insbesondere vorteilhaft ist, daß der relativ starke Berieselungsflüssigkeitsstrom, aufgrund der geforderten hohen Flüssigkeitsdurchsetzung, nicht direkt auf das oberste Lochblech bzw. die oberste Lochplatte 33 trifft und somit eine ungleichmäßige Verteilung des Berieselungsflüssigkeitsstromes hervorrufen könnte.

Ein Teil des Flüssigkeitsstromes 24 tritt durch die Zwischenräume 37 in den Raum zwischen oberer Platte 31 und oberer Lochplatte 33 ein und verteilt sich gleichmäßig, wonach er durch die Löcher nach unten fällt. Hierbei trifft er auf die oberste Gütercharge, dargestellt in Form von Flaschen 15, an denen er nach unten abfließt. Dabei trifft er auf die erste Beladeebene 11.

Der überwiegende Teil des Berieselungsflüssigkeitsstromes 24 tritt jedoch in den Raum zwischen äußerem Leitelement 39 und innerem Leitelement 35 ein und fällt senkrecht nach unten. Der so gebildete erste Strömungsweg vollzieht sich weiter zwischen dem äußeren Leitelement 23 und der inneren Trennwand 17 und teilt sich wiederum auf. Ein kleiner Teil der Berieselungsflüssigkeit tritt in den Bereich zwischen Trennwand 17 und innerem Leitelement 21 ein und wird so unterhalb der ersten Beladeebene 11 in den Raum zwischen Beladeebene 11 und Lochplatte 19 eingeführt. Die Lochquerschnitte des Lochblechs bzw. der Lochplatte 19 sind dabei so bemessen, daß weniger Flüssigkeit nach unten abfließt als von oben eintritt. Dies hat zur Folge, daß die Berieselungsflüssigkeit an der Oberkante 47 des inneren Leitelements 21, wie auch schon an der Oberkante 46 des inneren Leitelements 35, überläuft und sich weiter nach unten in dem Zwischenraum zwischen innerem Leitelement 21 und äußerem Leitelement 23 nach unten bewegt.

Durch die geneigten unteren Abschnitte 25 der äußeren Leitelemente 23 wird der Berieselungsflüssigkeitsstrom 24 nach innen umgelenkt, was dazu führt, daß der Flüssigkeitsstrom auf die Trennwand 17 gelenkt wird, an der er nach unten in den Bereich zwischen innerer Trennwand 17 und innerem Leitelement 21 fließt. Auch in dieser nächsten Beladeebene ist das Verhältnis der zugeführten Berieselungsflüssigkeitsmenge zum Lochquerschnitt der Lochplatte 19 so gewählt, daß wieder ein Teil der Berieselungsflüssigkeit überläuft und sich nach unten außerhalb der Beladeebene 11 bewegt.

In der in Fig. 1 gezeigten dritten Ladeebene - von oben gesehen - wird der Lochquerschnitt der Lochplatte 19 in bezug auf die heranströmende Berieselungsflüssigkeitsmenge 24 so gewählt, daß nahezu kein Flüssigkeitsstrom mehr übertritt, sondern sich ein konstanter Flüssigkeitspegel 49 einstellt. Selbstverständlich kann im Bedarfsfalle auch mit überlaufendem Flüssigkeitsstrom gearbeitet werden.

Der konstante Flüssigkeitspegel 49 hat jedoch den Vorteil, daß der gesamte eingeführte Flüssigkeitsstrom 24 über Berieselungsgut geführt wird und somit sich ein optimales Verhältnis von aufzuwendendem Flüssigkeitsstrom und zu berieselndem Gut ergibt. Dies ist insbesondere dann vorteilhaft, wenn, wie bei Sterilisation notwendig, eine unter Druck und hoher Temperatur stehende Berieselungsflüssigkeit mit großem Durchfluß durch die Berieselungskammer hindurchgeführt werden muß. Große nicht verwendete Flüssigkeitsmengen benötigen jedoch unnötigen Energieaufwand sowohl für die Aufheizung selbst als auch für die Umwälzung der Flüssigkeitsmengen.

Im Vorangegangenen wurde die Zufuhr des Berieselungsflüssigkeitsstromes 24 zu den einzelnen Beladeebenen dargestellt. Im Nachfolgenden wird nunmehr der Ablauf der Berieselungsflüssigkeit dargestellt, wie dies aus Fig. 2 deutlich wird. Die auf der ebenen Beladeebene 11 auftreffende Berieselungsflüssigkeit wird zu den jeweiligen Stirnseiten eines jeden Wagens 7 abgeführt und kann ungehindert von den Rändern der Stirnseiten nach unten fallen. Dadurch entsteht ein "Vorhang", der insbesondere an den beiden Stirnseiten der Kammer 3 den großen Vorteil hat, daß die doch lokal erheblich kühlere bzw. wärmere Stirnseite der Kammer durch den Flüssigkeitsvorhang abgedeckt wird, so daß die in den Randbereichen angeordneten zu berieselnden Güter wiederum mit gleicher Temperatur behandelt werden können, wie dies beispielsweise der Güter im Mittenbereich der Beladeebenen der Fall ist.

Da durch den Flüssigkeitsvorhang die gesamte jeweils einmal verwendete Berieselungsflüssigkeit abgeführt wird, ohne erneut mit zu berieselndem Gut in Berührung zu kommen, wird eine sehr gleichmäßige Verteilung der Temperatur in der Beladeebene erreicht und die eingangs genannten Nachteile herkömmlicher Berieselungsanordnungen vermieden.

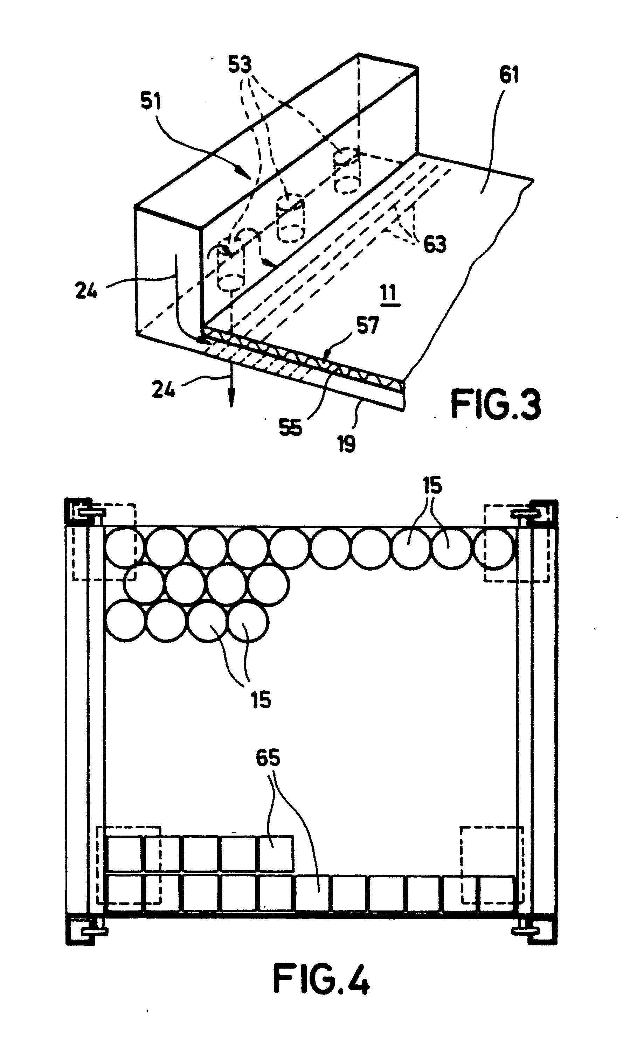

Gemäß Fig. 3 ist eine weitere Ausführungsform des seitlichen Zuführsystems der Berieselungsflüssigkeit 24 dargestellt. Dieses Zuführsystem weist eine Art zum Zwischenraum zwischen Beladeebene 11 und Lochplatte 19 offenen Auffangraum 51 auf, in dem die von oben eintretende Flüssigkeitsmenge aufgefangen und nach unten zur Lochplatte abgeführt wird. Durch den Überschuß an eintretendem Wasser füllt sich der Aufnahmeraum 51, in dem Stutzen 53 angeordnet sind. Durch diese Stutzen wird eine Mindestwassermenge in dem Aufnahmeraum 51 sichergestellt. Die oben offenen Stutzen 53 erlauben dann einen Überlauf der Berieselungsflüssigkeit 24, die dann nach unten in den darunterliegenden Aufnahmeraum 51 fällt.

Zur Vermeidung, daß die durch die Stutzen 53 fallende Flüssigkeit direkt durch Stutzen der darunterliegenden Beladeebene weiter nach unten geführt wird, sind die Stutzen einander benachbarter Beladeebenen 11 seitlich so versetzt angeordnet, daß die Berieselungsflüssigkeit 24 in einen Bereich neben den Stutzen 53 in den Aufnahmeraum 51 fällt.

Aus Fig. 3 ist weiterhin ein bevorzugter Aufbau der Beladeebene 11 dargestellt, wie er insbesondere für schwere Güter vorteilhaft ist. Die Beladeebene 11 weist eine wellenförmige Platte 55 auf, bei der vorteilhaft ist, daß das von oben auftreffende Wasser in den Senken 57 unterhalb des beispielsweise direkt auf die wellenförmige Platte 55 gesetzten Gutes ungehindert seitlich abfließen kann.

Zur Steigerung der Tragkraft kann unterhalb der wellenförmigen Platte 55, die auch zick-zackförmig ausgebildet werden kann, eine weitere Stützplatte 59 vorgesehen werden, die zu einer erheblichen Versteifung und damit Erhöhung der Tragfähigkeit der Beladeebene 11 führt. Um einen noch stabileren Aufbau der Beladeebene 11 zu bilden, kann weiterhin eine obere Stützplatte 61 vorgesehen sein, die sich auf der wellenförmigen Platte 55 befindet. Diese obere Stützplatte 61 weist sodann Öffnungen 63, in Fig. 3 nur schematisch angedeutet, auf, die einen Durchtritt der Berieselungsflüssigkeit bis hin zur wellenförmigen Platte 55 sicherstellen.

In Fig. 4 ist die Anordnung von zylindrischem bzw. quadratischem zu beaufschlagenden Gut dargestellt. Aufgrund der vorteilhaften homogenen Flüssigkeitszufuhr und Temperatur im gesamten Beladeraum ist es möglich, das Ladegut sehr eng aneinanderzustellen, so daß eine sehr hohe Flächenausnutzung möglich ist. Wie aus Fig. 4 deutlich, sind die Flaschen 15 entgegen herkömmlichen Beschickungsanordnungen versetzt angeordnet, d.h. es wird die maximale Ladedichte ausgenützt. Ebenso können quadratische Güter 65 sehr nahe aneinandergestellt werden und trotzdem wird ein optimales Arbeitsergebnis gewährleistet.

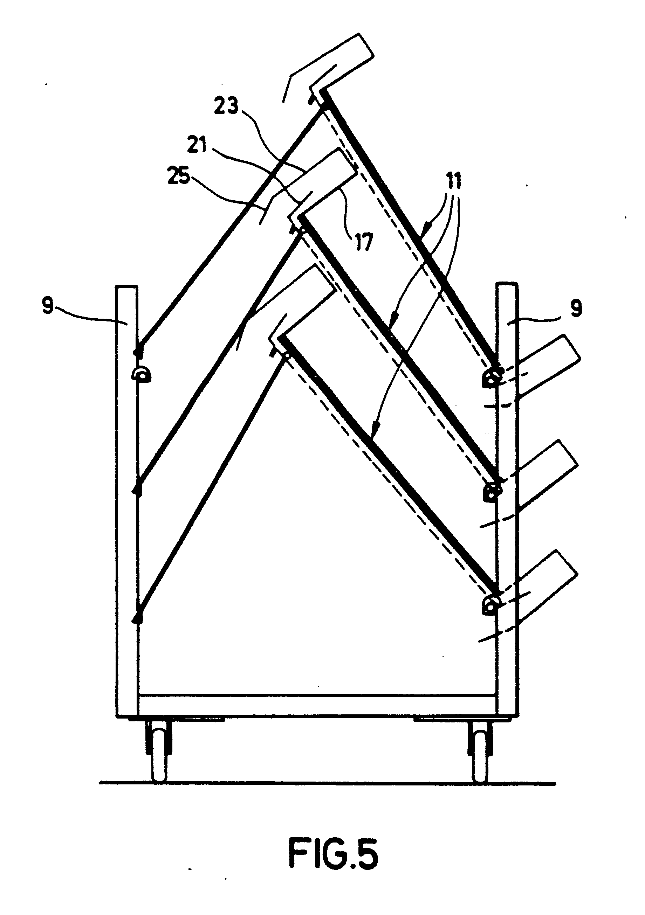

In Fig. 5 ist eine Möglichkeit der schwenkbaren Anordnung der Beladeebenen 11 dargestellt, welche eine leichtere Beschickung des Wagens erlauben, da Beladeebene für Beladeebene relativ frei zugänglich wird und somit das Ladegut leicht angeordnet werden kann.

Die Beladeebenen 11 sind vorteilhafterweise in ihrer Höhe im Wagen 7 verstellbar angeordnet. Hierzu sind sie auf einfache Weise auf Stangen oder Trägern gelagert, die ihrerseits in Löchern oder Ausnehmungen der Eckpfosten 9 gehalten sind. Dadurch wird erreicht, daß ohne zusätzliches Werkzeug der Abstand der Stangen verändert werden kann und somit die Höhe der beladenen Ebene zueinander verändert werden kann.

Die vorbeschriebene erfindungsgemäße Berieselungsanordnung schafft somit eine gleichmäßige Zufuhr der Berieselungsflüssigkeit für alle Bereiche des Ladeguts, wobei insbesondere durch die genannte Abführung der einmal verbrauchten Berieselungsflüssigkeit erreicht wird, daß durch die gleichmäßige Zufuhr eine gleichmäßige Temperaturverteilung im gesamten Beladeraum vorhanden ist, was zu einem hervorragenden Arbeitsergebnis, insbesondere bei Verwendung der Berieselungsanordnung für die Sterilisation führt.

## Ansprüche

1. Berieselungsanordnung zur Berieselung von Gütern in einem Beladeraum, die etagenweise übereinander auf Beladeebenen angeordnet sind, mit Berieselungsflüssigkeitszuführungen zur Abgabe der Berieselungsflüssigkeit an die Güter über eine Lochplatte,
**dadurch gekennzeichnet,**
daß oberhalb jeder Beladeebene (11) eine dieser individuell zugeordnete Lochplatte (19) vorgesehen ist,
daß außerhalb des Beladeraumes (5) für die Berieselungsflüssigkeit (24) jeder Beladeebene (11) gesondert zuführende erste Strömungswege vorgesehen sind, die jeweils die der Beladeebene (11) zugeordnete Lochplatte (19) mit für jede Beladeebene (11) gleicher Füllmenge beaufschlagen, und daß oberhalb jeder Lochplatte (19) mit Ausnahme der obersten (33) und untersten (34) Lochplatte ein die Berieselungsflüssigkeit (24) in im wesentlichen horizontaler Richtung zu wenigstens einer Seite des Beladeraumes (5) hin abführender zweiter Strömungsweg vorgesehen ist.

2. Berieselungsanordnung nach Anspruch 1, die eine Kammer (3) aufweist, in die ein die Beladeebene (11) aufweisender Wagen (7) einbringbar ist, **dadurch gekennzeichnet,** daß der erste Strömungsweg durch innere (21) und äußere (23) Leitelemente begrenzt wird.

3. Berieselungsanordnung nach Anspruch 2, **dadurch gekennzeichnet,** daß die Leitelemente (21, 23) sich im wesentlichen in vertikaler Richtung erstrecken.

4. Berieselungsanordnung nach Anspruch 3, **dadurch gekennzeichnet,** daß die inneren Leitelemente (21) an ihren unteren Enden mit dem zugeordneten Lochblech (19) verbunden sind, und gegenüber der Vertikalen entgegen der Strömungsrichtung leicht nach außen geneigt angeordnet sind.

5. Berieselungsanordnung nach einem der Ansprüche 2 bis 4, **dadurch gekennzeichnet,** daß die äußeren (23) und/oder die inneren (21) Leitelemente am Wagen (7) angebracht sind.

6. Berieselungsanordnung nach einem der Ansprüche 2 bis 5, **dadurch gekennzeichnet, daß** die äußeren Leitelemente in Strömungsrichtung der Berieselungsflüssigkeit (24) mit unteren Abschnitten (25) nach innen vorspringen.

7. Berieselungsanordnung nach einem der Ansprüche 1 bis 6, **dadurch gekennzeichnet, daß** die Beladeebenen (11) in vertikaler Richtung verstellbar angeordnet sind.

8. Berieselungsanordnung nach einem der Ansprüche 1 bis 7, **dadurch gekennzeichnet, daß** die Berieselungsflüssigkeit (24) und der Lochquerschnitt der Löcher der Lochplatte (19) so aufeinander abgestimmt ist, daß eine von Beladeebene zu Beladeebene abnehmende Berieselungsflüssigkeitsmenge überläuft und sich bevorzugt in der untersten Beladeebene ein im wesentlichen konstanter Berieselungsflüssigkeitspegel (49) einstellt.

9. Berieselungsflüssigkeit nach einem der Ansprüche 1 bis 8, **dadurch gekennzeichnet, daß** die oberste Lochplatte (33) von einer Platte (31) nahezu vollständig übe rdeckt ist und der Flüssigkeitseintritt an einander gegenüberliegenden Seiten erfolgt.

10. Berieselungsanordnung nach einem der Ansprüche 1 bis 9, **dadurch gekennzeichnet, daß** die Beladeebene (11), auf der die Güter (65) ruhen, aus einer wellenförmigen (55) oder zick-zack-förmigen Platte gebildet ist.

11. Berieselungsanordnung nach Anspruch 10, **dadurch gekennzeichnet, daß** das Wellen- oder Zick-Zack-Profil zwischen den beiden Längsseiten der Kammer (3) verläuft.

12. Berieselungsanordnung nach Anspruch 10 oder 11, dadurch gekennzeichnet, daß unterhalb der profilierten Platte (55) eine im wesentlichen ebene Stützplatte (59) angeordnet ist.

13. Berieselungsanordnung nach einem der Ansprüche .10 bis 12, **dadurch gekennzeichnet,** daß über der profilierten Platte (55) eine im wesentlichen ebene obere Stützplatte (61) angeordnet ist, die im Bereich der Senken (57) der profilierten Platte Öffnungen (63) für den Durchtritt der Berieselungsflüssigkeit (24) aufweist.

14. Berieselungsanordnung nach einem der Ansprüche 1 bis 13, **dadurch gekennzeichnet,** daß die abfließende Berieselungsflüssigkeit an den Stirnseiten der Beladeebenen (11) einen Flüssigkeitsvorhang bildet.

15. Berieselungsanordnung nach Anspruch 1, **dadurch gekennzeichnet,** daß die Zuführung der Berieselungsflüssigkeit (24) über Aufnahmeräume (51) erfolgt, in denen Stutzen (53) angeordnet sind, die ein Ablaufen der Berieselungsflüssigkeit (24) nach unten erlauben.

16. Berieselungsanordnung nach Anspruch 15, **dadurch gekennzeichnet, daß** die Überlaufstutzen (53) einer Beladeebene (11) bezüglich der benachbarten Beladeebenen versetzt angeordnet sind.

17. Berieselungsanordnung nach einem der Ansprüche 7 bis 16, **dadurch gekennzeichnet,** daß die Verstellbarkeit der Beladeebenen (11) durch Stangen erreicht wird, auf denen die Beladeebenen (11) ruhen, und daß die Stangen ihrerseits in Löchern von Eckpfosten (9) des Wagens (7) gelagert sind.

18. Berieselungsanordnung nach einem der Ansprüche 1 bis 17, **dadurch gekennzeichnet,** daß die Beladeebenen (11) seitlich schwenkbar angeordnet sind.

FIG.1

FIG.2

FIG.3

FIG.4

FIG.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 288 481 (F. HANAU)<br>* Seite 12, Zeile 39 - Seite 14, Zeile 16; Figuren 4-12 *<br>--- | 1 | B 65 B 55/10<br>A 61 L 2/04<br>A 23 L 3/10 |
| A | FR-A-2 374 078 (FMC)<br>--- | | |
| A | FR-A-2 290 249 (FMC)<br>--- | | |
| A | US-A-4 085 668 (FMC)<br>----- | | |

RECHERCHIERTE SACHGEBIETE (Int. Cl.5)

B 65 B
A 61 L
A 23 L

Der vorliegende Recherchenbericht wurde für alle Patentansprüche erstellt

| Recherchenort | Abschlußdatum der Recherche | Prüfer |
|---|---|---|
| DEN HAAG | 04-09-1990 | JAGUSIAK A.H.G. |